Europäisches Patentamt

European Patent Office

Office européen des brevets

(11) Veröffentlichungsnummer: **0 406 732 B1**

(12) **EUROPÄISCHE PATENTSCHRIFT**

(45) Veröffentlichungstag der Patentschrift: **24.05.95**

(21) Anmeldenummer: **90112513.8**

(22) Anmeldetag: **30.06.90**

Die Akte enthält technische Angaben, die nach dem Eingang der Anmeldung eingereicht wurden und die nicht in dieser Patentschrift enthalten sind.

(51) Int. Cl.⁶: **A61K 35/42**, A61K 31/685, C07K 2/00

(54) **Niedrig-viskose, hochkonzentrierte Surfactant-Suspension.**

(30) Priorität: **04.07.89 DE 3921954**

(43) Veröffentlichungstag der Anmeldung: **09.01.91 Patentblatt 91/02**

(45) Bekanntmachung des Hinweises auf die Patenterteilung: **24.05.95 Patentblatt 95/21**

(84) Benannte Vertragsstaaten: **AT BE CH DE DK ES FR GB GR IT LI LU NL SE**

(56) Entgegenhaltungen:
EP-A- 0 110 498
EP-A- 0 119 056
EP-A- 0 145 005
EP-A- 0 286 011
GB-A- 2 050 832

Biological abstract nb: 83008933; DAVIES R.J. et al: "The behaviour of lung surfactant in electrolyte solutions."

Biological abstract nb: 85052770; EFRATI H. et al: "Divalent cation and hydrogen ion effects on the structure and the surface activity of pulmonary surfactant."

(73) Patentinhaber: **Dr. Karl Thomae GmbH**

**D-88397 Biberach (DE)**

(72) Erfinder: **Disse, Bernd, Dr.Dr. Dipl.-Chem. Albrecht-Dürer-Strasse 17 D-6507 Ingelheim/Rhein (DE)**
Erfinder: **Weller, Eberhard Volmarweg 44 D-7950 Biberach 1 (DE)**
Erfinder: **Becker, Robert, Dr. Dipl. Chem. Hühnerfeldstrasse 26 D-7950 Biberach 1 (DE)**

EP 0 406 732 B1

Biological abstract nb: 79099919; HAWGOOD S. et al: "Effects of a surfactant-associated protein and calcium ions on the structure and the surface activity of lung surfactant lipids."

Biological abstract nb: 85021943; HAAGS-MAN H.P. et al : "The major lung surfactant protein SP-28-36 is a calcium-dependent carbohydrate-binding protein."

BRITISCH MED. J. 294: 991-996 (1987)

ACTA PAEDIATR. SCAND. 76: 697-705, 1987

LIPIDS, VOL. 18, NO. 8, 522-529 (1983)

**Beschreibung**

Die Erfindung betrifft niedrig-viskose, hochkonzentrierte Surfactant-Suspensionen zur Substitutionstherapie bei Atemwegserkrankungen und Verfahren zu ihrer Herstellung. Bei dem Surfactant handelt es sich um ein oberflächenaktives Aggregat aus Phospholipiden, Neutrallipiden und Surfactant-assoziierten hydrophoben Proteinen, gewonnen aus isoliertem, natürlichem pulmonalem Surfactant; das Surfactant kann aber auch aus synthetischen Quellen stammen, hierbei setzt es sich zusammen aus synthetischen Lipiden und, gegebenenfalls, rekombinanten Proteinen.

Ein Mangel an pulmonalem Surfactant ist als primäre Ursache des Atemnotsyndroms unreifer Frühgeborener erkannt worden (Avery ME, Mead J. Surface properties in relation to atelectasis and hyaline membrane disease. Amer J Dis Child 97: 517-523 (1959)). Der Nutzen einer Substitutionstherapie mit natürlichem Surfactant ist aus experimentellen und klinischen Studien bekannt (Van Golde, LMG, Batenburg JJ, Robertson B. The pulmonary surfactant system: biochemical aspects and functional significance. Physiol Rev 68: 374-455 (1988), Robertson B, Lachmann B. Experimental evaluation of surfactants for replacement therapy. Experimental Lung Research 14: 279-310 (1988)). Abhängig von der Aktivität des verwendeten Surfactants oder von der Einschätzung der Autoren wurden Einzeldosen zwischen 50 und 200 mg/kg intratracheal instilliert. Das Atemnotsyndrom erwachsener Patienten besitzt gewisse Parallelen zum Atemnotsyndrom unreifer Frühgeborener. Nach Meinung zahlreicher Wissenschaftler bietet Surfactantsubstitution bei diesem Krankheitsbild mit hoher Mortalität eine therapeutische Chance (Lachmann B, Surfactant replacement in acute respiratory failure: Animal studies and first clinical trials, in B. Lachmann ed.: Surfactant replacement therapy in neonatal and adult respiratory distress syndrome. Springer-Verlag, Berlin 1988 pp 212-223). Für diesen Therapieansatz werden hohe Dosierungen bis 400 mg/kg vorgeschlagen (Lachmann B, Surfactant replacement in acute respiratory failure: Animal studies and first clinical trials, in B. Lachmann ed.: Surfactant replacement therapy in neonatal and adult respiratory distress syndrome. Springer-Verlag, Berlin 1988 pp 212-223; Spragg RG, Richmann P, Gillard N, Merritt TA. The future for surfactant therapy of the adult respiratory distress syndrome, in B. Lachmann ed.: Surfactant replacement therapy in neonatal and adult respiratory distress syndrome. Springer-Verlag, Berlin 1988 pp 203-211).

Bei allen Anwendungen stellt die Belastung der Lunge des Patienten mit hohen Flüssigkeitsmengen ein Problem dar. Enhorning hat seine frühgeborenen Patienten mit 100 mg/kg Surfactant als 25 mg/ml Suspension therapiert. Dies bedeutet immerhin die Gabe von 4 ml Flüssigkeit pro kg Körpergewicht (Enhorning G. Shennan A, Possmayer F, Dunn M, Chen CP, Milligan J. Prevention of neonatal respiratory distress syndrome by tracheal instillation of surfactant: A randomized clinical trial. Pediatrics 76: 145-153 (1985)). Robertson hat Einzeldosen von 200 mg/kg relativ hochkonzentrierter Suspension von 80 mg/ml verabreicht (Noack G, Bergren P, Curstedt T, Grossmann G, Herin P, Mortensson W, Nilsson R, Robertson B, Severe neonatal respiratory distress syndrome treated with the isolated phospholipid fraction of natural surfactant. Acta Paediatr Scand 76: 697-705 (1987)). Dies bedeutet die Gabe von 2,5 ml/kg. Die höchsten Konzentrationen verwendete Morley mit synthetischem Surfactant (Ten centre study group. Ten centre trial of artificial surfactant (artificial lung expanding compound) in very premature babies. British Med J 294: 991-996 (1987)). Hier wurden 100 mg/kg als 100 mg/ml Kristallsuspension gegeben. Allerdings waren bis zu 400 mg/kg in 24 Stunden notwendig. Relativ günstige Verhältnisse wurden mit einem Surfactant aus einem oberflächenaktiven Aggregat aus Phospholipiden, Neutrallipiden und Surfactant-assoziierten hydrophoben Proteinen, gewonnen aus isoliertem, natürlichem Surfactant aus Rinderlungen, im folgenden SF-RI I genannt, erreicht. Es wurden 50 mg/kg als 40 mg/ml Suspension gegeben. Dies bedeutet eine Belastung der Patienten mit einem Volumen von 1,2 ml/kg.

Transferiert man die Verhältnisse auf die Therapie des Erwachsenen, so könnte eine Dosis von 200 mg/kg, gegeben als 40 mg/ml Suspension immerhin bedeuten, daß einem Patienten von 70 kg Körpergewicht 350 ml intratracheal instilliert werden müßten. Diese Berechnung zeigt, daß es wünschenswert ist, die Konzentration des Surfactant möglichst hoch zu wählen. Begrenzt wird dieser Wunsch durch die hohe Viskosität von Surfactantsuspensionen über 60 mg/ml. Eine hohe Viskosität führt zu Problemen bei der Applikation und Verteilung des Surfactant und führt zum Anstieg der Atemwegsresistance. Robertson erreichte 80 mg/ml, imdem er aus einem aus Schweinelungen gewonnenen Surfactant die Neutrallipide abtrennte (Noack G, Bergren P, Curstedt T, Grossmann G, Herin P, Mortensson W, Nilsson R, Robertson B, Severe neonatal respiratory distress syndrome treated with the isolated phospholipid fraction of natural surfactant. Acta Paediatr Scand 76: 697-705 (1987)). Morley verwendete 100 mg/ml Kristallit-Suspension von synthetischen Lipiden. British Med J 294: 991-996 (1987)). Er erkaufte die hohe Konzentration mit dem Zwang zur Verwendung eisgekühlter Suspendiermittel.

Überraschenderweise wurde nun gefunden, daß auch bei Surfactant-Einwaagen von >60 mg/ml und Lagerung bei Raumtemperatur über Stunden die Viskosität der Suspensionen unter 30 mPas bleibt, sofern

a.) das verwendete Surfactant-Lipid/Protein-Präparat einen Gehalt an an dieses gebundenen $Ca^{2+}$ und/oder $Mg^{2+}$-Ionen von mehr als 25 mmol/mol, vorzugsweise mehr als 40 mmol/mol organisch extrahierbarer Phospholipide enthält,

b.) das Suspendiermittel mindestens 1 mmol/l, vorzugsweise 2,5 bis 3,5 mmol/l $Ca^{2+}$ und/oder $Mg^{2+}$-Ionen und

c.) das Suspendiermittel gleichzeitig mindestens 33 mmol/l, vorzugsweise 60 bis 150 mmol/l Natriumchlorid enthält.

Als Suspendiermittel wird im allgemeinen Wasser verwendet. Fehlt nur einer dieser Faktoren oder ist nur einer dieser Faktoren mit einer zu geringen Konzentration vorhanden, so steigt die Viskosität der so gewonnenen Suspensionen bei Raumtemperatur dramatisch an. Bei Einhaltung aller drei Faktoren kann selbst aus einem Vesikel-Lyophilisat ein niedrig viskoses Surfactant von hoher Konzentration hergestellt werden, das auch bei Raumtemperatur mehr als 12 Stunden stabil ist. Eine derartige Surfactantsuspension zeigt auch bei sehr hohem Surfactantgehalt keinen gefährlich werdenden Anstieg der Atemwegsresistance und eignet sich bestens zur Anwendung oder Substititutionstherapie bei Atemwegserkrankungen. Eine derartige Surfactantsuspension gestattet die intratracheale Zufuhr des Surfactants in hoher Dosierung, ohne daß die Lunge mit Wasser überlastet ist oder sonstige negative Beeinträchtigungen, z. B. durch Anstieg der Viskosität, erfährt. Während die bisher bekannten Surfactant-Suspensionen bereits ab 60 mg Surfactant pro ml sehr viskos sind, ist es jetzt durch das erfindungsgemäße Vorgehen möglich, Suspensionen in Konzentrationen von 200 mg/ml anzufertigen; für den therapeutischen Einsatz sehr geeignet sind z. B. Surfactant Konzentrationen um 150 mg/ml Suspension. Surfactant-Suspensionen geringerer Viskosität können, falls dies gewünscht wird, leicht dadurch erhalten werden, daß die erfindungsgemäßen Suspensionen mit erfindungsgemäßen Suspendiermitteln nach Belieben verdünnt werden.

Die Beladung der organisch extrahierbaren Phospholipide nach ihrer Isolierung aus den Lavagen der Lungen von beispielsweise Rind oder Schwein mit $Ca^{2+}$ und/oder $Mg^{2+}$-Ionen erfolgt dadurch, daß bei der Extraktion der wäßrigen Phase mit z. B. Chloroform die wäßrige Phase mit einem $Ca^{2+}$ bzw. $Mg^{2+}$-Ionenüberschuß versehen wird. In Abhängigkeit von der Art der Aufarbeitung ergibt sich eine Obergrenze der $Ca^{2+}$ bzw. $Mg^{2+}$-Ionen von zirka 70 mmol $Ca^{2+}$ bzw. $Mg^{2+}$/mol Phospholipid. Hat ein Phospholipidgemisch oder Phospolipid-Proteingemisch eine zu geringe Beladung mit $Ca^{2+}$ bzw. $Mg^{2+}$-Ionen wie z. B. das 13 mmol $Ca^{2+}$/mol Phospholipid-Proteingemisch in der Charge B (Tabelle 1), so kann diese Charge nachträglich in organischen Lösungen mit den fehlenden $Ca^{2+}$ bzw. $Mg^{2+}$-Ionen beladen werden. Dies geschieht z. B. durch Zugabe von $CaCl_2$, welches in Methanol gelöst ist. Generell läßt sich sagen, daß sich alle bekannten Trockenlipide mit $Ca^{2+}$ und/oder $Mg^{2+}$-Ionen beladen und zu Suspensionen mit geringer Viskosität verarbeiten lassen.

Die Calcium- und Magnesiumionen werden in Form löslicher Calcium- und Magnesiumsalze zugeführt. Man verwendet hierzu vorzugsweise Calciumchlorid bzw. Magnesiumchlorid; man kann aber auch andere physiologisch verträgliche, wasserlösliche Calcium- und/oder Magnesiumsalze hierzu verwenden, so z. B. Calcium- oder Magnesiumsulfat, Calciumnitrat, Calciumlactat, Calciumsorbat, Caliciumascorbat, Calciumgluconat, Calciumlactogluconat, Magnesiumhydrogencitrat, Magnesiumdihydrogenglutamat, Magnesiumcitrat.

Die Herstellung des Surfactant-Materials als Ausgangsprodukt für die niedrig-viskosen, hochkonzentrierten Surfactant-Suspensionen erfolgt in Anlehnung an die von Yu et al. (Lipids 18: 522-529 (1983)) beschriebene Methode. Eine wesentliche Abweichung von dieser Methode ist die Verwendung von Calcium- bzw. Magnesium-haltigen Lösungsmitteln oder Suspendiermitteln während des ganzen Prozesses. Rinderlungen werden mit einer Kochsalzlösung, die 3 mmol/l $Ca^{2+}$ und/oder $Mg^{2+}$-Ionen enthält, ausgewaschen. Die Abzentrifugation des Surfactants erfolgt aus einer Lösung, die 6 bis 12 mmol/l $Ca^{2+}$ und/oder $Mg^{2+}$-Ionen, vorzugsweise $Ca^{2+}$-Ionen, enthält. Die Aufreinigung über eine Dichtegradientenzentrifugation mit Glucose bei einer Dichte zwischen 1,05 und 1,15 g/ml erfolgt wiederum in Gegenwart von 3 bis 8 mmol/l $Ca^{2+}$ und/oder $Mg^{2+}$-Ionen. Der Aufreinigung schließt sich eine Reinigung durch Verteilung im System Chloroform-Methanol-Wasser nach Bligh und Dyer (vgl. Can. J. Biochem. Physiol. 37: 911-917 (1959)) unter Verwendung einer wäßrigen Phase mit 3 bis 10 mmol/l $Ca^{2+}$ und/oder $Mg^{2+}$-Ionen, vorzugsweise 6 mmol/l, an. Die organische Phase wird anschließend bei Raumtemperatur im Vakuum eingedampft und der Rückstand getrocknet und schließlich zerkleinert. Alternativ kann die organische Phase auch gefriergetrocknet werden. In jedem Fall ist das gewonnene Produkt über längere Zeit (mindestens 3 Jahre bei Kühlschranktemperatur) lagerstabil. Das so erhaltene feinpulvrige trockene Lipid wird in einer wäßrigen Lösung, die die erfindungsgemäßen Mengen an Kochsalz und an $Ca^{2+}$ und/oder $Mg^{2+}$-Salzen enthält, durch kräftiges Schütteln bis zur Erreichung der gewünschten Lipidkonzentration suspendiert. Auch eine so erhaltene Suspension ist für einige Tage Kühlschranktemperaturen lagerfähig und kann bei Bedarf intratracheal appliziert werden.

Das Suspendiermittel soll mehr als 1 mmol/l, vorzugsweise 2,5 bis 3,5 mmol/l, $Ca^{2+}$ und/oder $Mg^{2+}$-Ionen und mehr als 33 mmol/l, vorzugsweise 75 mmol/l und höchstens 150 mmol/l, Kochsalz enthalten.

Viskositätsuntersuchungen mit verschiedenen Surfactant-Zubereitungen.

Material und Methoden:

Für die nachfolgenden Untersuchungen wurde als Ausgangsmaterial ein extrahiertes Surfactant aus Lavage von Rinderlungen verwendet (im folgenden SF-RI I genannt). Das Verfahren stellt in den Grundzügen eine Modifikation und Optimierung des Verfahrens von Yu et. al dar (Yu S, Harding PGR, Schmith N, Possmayer F. Bovine pulmonary surfactant: chemical composition and physical properties. Lipids 18: 522-529 (1983)). Wesentliche Abweichungen von dieser Arbeit waren die Verwendung von calcium-haltigen Lösemitteln während des ganzen Prozesses: Die Auswaschung von Rinderlungen wurde mit einer Kochsalzlösung vorgenommen, die Lavage-Flüssigkeit wurde auf einen Calciumgehalt von 6 mmol/l eingestellt (mit $CaCl_2$). Die Abzentrifugation von Surfactant erfolgte aus einer Lösung, die 9 mmol/l Calcium enthielt. Die Aufreinigung über einen Glukose-Dichtegradienten erfolgte bei Anwesenheit von 6 mmol/l Calcium. Der wichtigste Schritt war, daß die Reinigung durch Verteilung im System Chloroform-Methanol-Wasser nach Bligh and Dyer (Can. J. Biochem. Physiol. 37: 911-913 (1959)) ebenfalls unter Verwendung einer wäßrigen Phase, die 6 mmol/l Calciumchlorid enthielt, vorgenommen wurde.

Nach erfolgter Phasentrennung wurde die organische Phase bei Raumtemperatur zur Trocknung einrotiert und anschließend im Vakuum getrocknet. Der Wassergehalt nach Karl Fischer sollte möglichst unter 3 % liegen. Durch Sieben wurde das Material auf eine Partikelgröße von ca. 160 $\mu$m verkleinert.

Für die Untersuchungen wurde dieses feinpulvrige trockene Lipid in wäßrigen Lösungen mit unterschiedlichen Gehalten an Calciumchlorid und Natriumchlorid in Ampullenwasser durch kräftiges Schütteln suspendiert. Die Konzentration der Suspensionen lag zwischen 60 und 250 mg/ml.

Die Viskosität dieser Suspensionen wurde nach Lagerung bei Raumtemperatur in bestimmten Zeitabschnitten mit Hilfe eines Kapillarviskosimeters bestimmt. Es handelt sich hierbei um ein modifiziertes kapillarviskosimetrisches Meßverfahren, beschrieben von Weller et al. in Wiener Medizinische Wochenschrift suppl. 33: 7-12 (1975) und von Weiler in Viskosimetrie inhomogener Flüssigkeiten, Dustri Verlag München, pp 21-41. Es wird nachfolgend kurz erläutert. Die in einer Injektionsspritze befindliche Probe wird mit definierter konstanter Geschwindigkeit durch eine Meßkapillare gedrückt. Auch Inhomogenitäten sind feststellbar. Die Schergeschwindigkeit ist bekannt und konstant. Die Kalibration erfolgt mit Eichölen. Der Druck vor der Kapillare ist ein Maß für die Viskosität und wird mit einem Schreiber fortlaufend registriert.

Gegen Ende des Untersuchungszeitraumes wurden einige Suspensionen durch Eintrag von Ultraschallenergie auch in Vesikelsuspensionen umgewandelt (Branson-Ultraschallgerät, Mikrospitze, 20 Watt, 2 min/ml, Kühlung mit Eiswasser).

Ebenfalls untersucht wurden Suspensionen, die durch Aufschütteln von gefriergetrockneten Lösungen oder Vesikel-Dispersionen hergestellt worden waren. Hierzu wurde Surfactant soweit notwendig durch Eintrag von Ultraschallenergie in Ampullenwasser in Konzentrationen von ca. 40 mg/ml dispergiert oder, in anderen geeigneten Lösungsmitteln, wie z. B. tert.-Butanol, in Konzentrationen von ca. 40 bis 250 mg/ml gelöst. Die Suspensionen bzw. Lösungen wurden anschließend gefriergetrocknet. Der sich bildende Kuchen wurde zerstoßen und das resultierende Pulver weiter verarbeitet.

Auch Varianten des Herstellungsprozesses für SF-RI I wurden in die Untersuchungen einbezogen. Die Surfactantcharge B (siehe Tabelle 1) wurde ohne Zusatz von Calciumchlorid in den Auswasch- und Reinigungsmedien hergestellt.

Die Bestimmung von Calcium erfolgte durch Atomabsorptionsphotometrie nach Verbrennung geeigneter verdünnter Suspensionen von Surfactant in einer Acetylenflamme bei etwa 3000°C (Zeiss FL6). Bei diesen Temperaturen wird auch in einer Proteinmatrix gebundenes Calcium freigesetzt und richtig erfaßt.

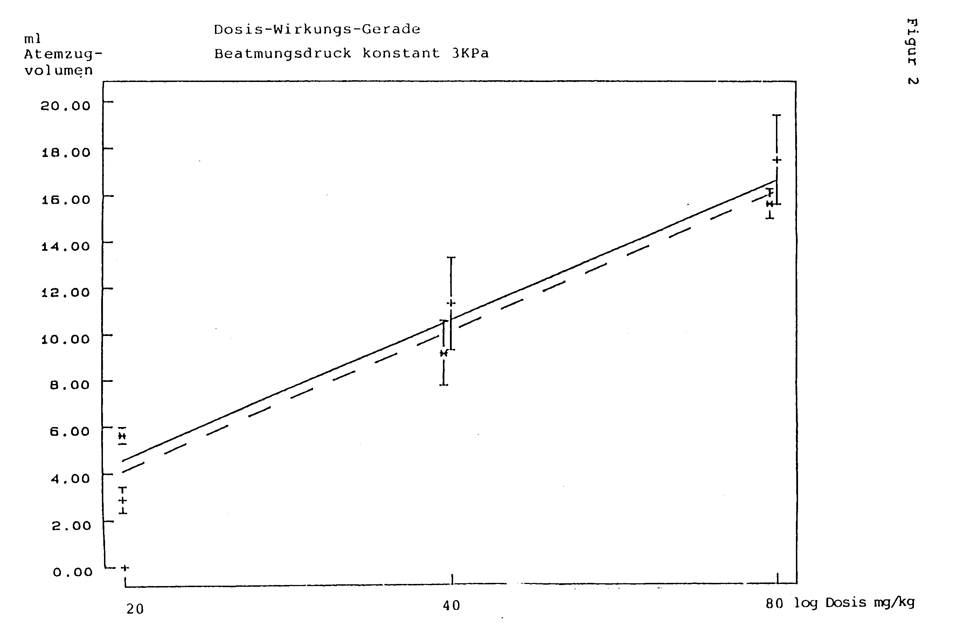

Die Bestimmung der relativen Wirksamkeit der Surfactant-Suspensionen an unreifen Kaninchenfeten erfolgte über den Vergleich mit einem Bezugsstandard. Dabei wird für die zu prüfende Charge und für den Bezugstandard je eine Dosis-Wirkungs-Kurve mit 3 Dosierungen erstellt (vgl. Figur 2). Mit Hilfe allgemein bekannter statistischer Kriterien (Kovarianzanalyse) wurde nachgewiesen, daß die erfindungsgemäße hochkonzentrierte Suspenion mit $Ca^{2+}$-Ionen von der niedrigkonzentrierten Vesikel-Dispersion bei gleicher Dosierung in ihrer Wirkung nicht signifikant verschieden war.

Ergebnisse und Diskussionen:

Der Calciumgehalt repräsentativer Chargen von SF-RI I ist in Tabelle 1 wiedergegeben.

Tabelle 1

Viskosität einer Suspension von 150 mg Surfactant pro ml Suspension

| Zube-reitung | Ca²⁺-Gehalt [mmol/mol] | Suspensions-medium CaCl₂ [mmol/l] | NaCl [mmol/l] | Lagerung bei Raumtemperatur bis (zwischen Zubereitung der Suspension und Messung der Viskosität) [mPas] | | | | | | | | |
|---|---|---|---|---|---|---|---|---|---|---|---|---|
| | | | | 5 min | 10 min | 15 min | 30 min | 45 min | 60 min | 90 min | 120 min | 16 h |
| A | 48 | 3 | 0 | | 304 | | 295 | | | | | |
| A | 48 | 0 | 75 | 11 | 20 | 20 | 34 | 37 | 118 | 148 | | |
| A | 48 | 1 | 75 | 9 | | 15 | 22 | | 113 | | | |
| A | 48 | 3 | 75 | 7 | 7 | 8 | 9 | | 13 | | | |
| A' | 58 | 3 | 75 | | 21 | 22 | 24 | | | 22 | 24 | |
| B | 13 | 3 | 75 | | 40 | 56 | 160 | | 177 | | 176 | |
| B | 13 | 12 | 75 | | 13 | | 25 | | 40 | | 60 | |
| C | 42 | 0 | 75 | | 181 | 184 | | | 202 | 219 | | |
| C | 42 | 3 | 75 | 6 | 8 | | 9 | 14 | 16 | | | 21 |
| C | 42 | 3 | 75 | Viskosität nach Beschallung 2 min/ml, 20 Watt Branson Beschaller mit Mikrospitze: 222 mPas | | | | | | | | |

A : pulverförmiges Surfactant, 160 μm Teilchengröße, Herstellung wie im Text beschrieben durch Modifikation des Verfahrens von Yu et al., Lipids 18, 522-528 (1983),

A': pulverförmiges Surfactant, nicht definierte Teilchengröße, Herstellung wie bei A,

B : pulverförmiges Surfactant, nicht definierte Teilchengröße, Herstellung wie bei A,

Herstellung wie bei A, jedoch ohne Ca²⁺-Zusatz während des Aufarbeitungsverfahrens,

C : lyophilisiertes Surfactant, Herstellung des Lipid-Proteingemisches wie bei A.

Die Ergebnisse zeigen, daß alle Chargen, die dem geschilderten Produktionsverfahren entsprechen, Gehalte an gebundenem Calcium von mehr als 40 mmol/mol lipidextrahierbaren Phosphats enthalten. Die ohne Zusatz von Calcium aufgearbeitete Charge (B) enthält hingegen nur 13 mmol/mol. Sie liegt damit im Bereich publizierter Werte für Calciumgehalte in extrahiertem pulmonalem Surfactant, die 16 mmol/mol nicht überschreiten (Weber MJ, Possmayer F. Calcium interactions in pulmonary surfactant. Biochim Biophys Acta 796: 83-91 (1984)). Wie sich nachfolgend zeigt, sind diese hohen Gehalte an gebundenem Calcium für die Eigenschaften von SF-RI I sehr wichtig.

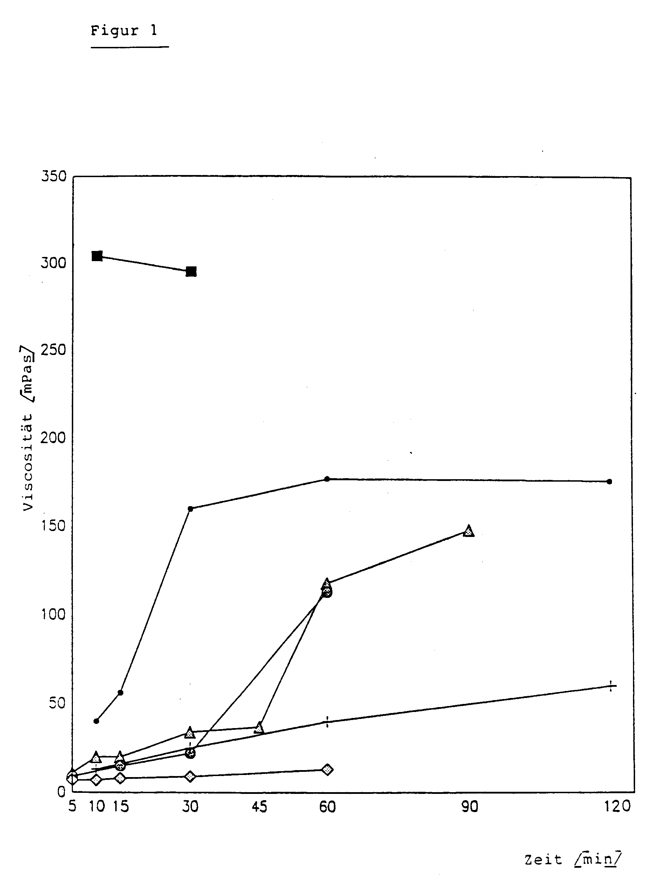

Tabelle 1 und Figur 1 enthält auch die Ergebnisse der Untersuchungen der Viskosität von frisch hergestellten Surfactant-Suspensionen im Zeitverlauf. Wenn ein "calcium-armes", also literaturübliches Surfactant (B), in einer Lösung aus 75 mmol/l Kochsalz und 3 mmol/l Calciumchlorid suspendiert wurde, dann stieg bei Raumtemperatur die Viskosität rasch an und überschritt bereits nach 30 Minuten 150 mPas. Dies bedeutet, daß die Surfactantsuspension eine sahneförmige Konsistenz angenommen hatte. Diese Suspension ist klinisch nicht für intratracheale Instillationen verwendbar.

Wenn ein Surfactant mit hohem gebundenem Calciumgehalt (A, A′ und C) in einer Lösung suspendiert wurde, die 3 mmol/l Calciumchlorid enthielt, so stieg die Viskosität ebenfalls sehr rasch an. Auch wenn in einer Kochsalzlösung von 75 mmol/l suspendiert wurde, so erreichte die Viskosität nach 90 Minuten Lagerung bei Raumtemperatur annähernd 150 mPas. Suspendierung in einer Lösung von 75 mmol/l Kochsalz plus 1 mmol/l Calciumchlorid führte zu einer langsamen und verminderten viskositätszunahme.

Sehr befriedigende Ergebnisse wurden durch Suspendierung von "calcium-reichem" Surfactant in einer Lösung von 75 mmol/l Kochsalz plus 3 mmol/l Calciumchlorid erreicht: Hier überstieg die Viskosität auch nach langer Lagerung nicht 25 mPas. Vergleichbare Ergebnisse wurden durch Suspendierung von "calcium-reichen" Surfactant-Lyophilisaten in einer Lösung von 75 mmol/l Kochsalz plus 3 mmol/l Calciumchlorid erzielt (Charge C). Auch hier überstieg die Viskosität sogar nach langer Lagerung bei Raumtemperatur für 16 Stunden 25 mPas nicht. Erst nach Eintrag von Ultraschallenergie stieg die Viskosität auf 220 mPas an.

Suspendierung des "Ca-armen" Lipids (B) in einer Lösung mit einer unphysiologisch hohen Ca-Konzentration von 12 mmol/l plus 75 mmol/l NaCl bewirkten eine verlangsamte und verminderte Viskositätszunahme. Jedoch wurde nach 120 Minuten immer noch ein Wert von 60 mPas erreicht. Die Konzentration 12 mmol/l war so berechnet, daß die eingestellte Endkonzentration (Summe aus $Ca^{2+}$-Gehalten im Suspendiermittel und im Lipid) auf denselben Wert kommt wie bei Suspendierung von A, A′ oder C in 3 mmol/l Ca-haltiger Lösung. Diese Variante, hohe Ca-Gehalte im Suspendiermittel vorzusehen, ist im Ergebnis weniger günstig und bei vorgesehener in vivo Anwendung nicht anwendbar, weil unphysiologisch hohe Ca-Konzentrationen im Suspendiermittel zur Anwendung kommen, die eine Bronchokonstriktion auslösen können.

Die Ergebnisse zeigen, daß überraschenderweise für die Erzielung hochkonzentrierter und trotzdem gering visköser Surfactant-Suspensionen drei Faktoren unentbehrlich sind: Das verwendete Surfactant-Lipid/Protein-Präparat muß einen hohen Gehalt an gebundenem Calcium aufweisen, vorzugsweise mehr als 25 mmol/mol organisch extrahierbarer Phospholipide. Das Suspendiermittel muß sowohl NaCl als auch $CaCl_2$ gelöst enthalten, vorzugsweise in einer Konzentration von 75 mmol/l, bzw. 3 mmol/l. Die Untersuchungen in Tabelle 1 zeigen, daß bei Fehlen auch nur einer dieser Faktoren die Viskosität der Suspensionen bei Raumtemperatur nach kurzer Zeit drastisch anstieg. Bei Einhaltung aller drei Faktoren jedoch konnte selbst aus einem Vesikel-Lyophilisat ein niedrigvisköses Surfactant von hoher Konzentration hergestellt werden, das auch bei Raumtemperatur mehr als 12 Stunden stabil war. Diese Art von Surfactantsuspension ist für therapeutische Anwendungen geeignet. Die Figur 1 zeigt nochmals den Verlauf der Viskosität einer Suspension mit 150 mg Surfactant pro ml in Abhängigkeit der $Ca^{2+}$-Konzentration gebunden an Surfactant und gelöst im Suspendiermittel mit und ohne Kochsalzzusatz.

Die atemphysiologische Wirksamkeit von Surfactant kann an unreifen Kaninchenfeten untersucht werden (Robertson B, Lachmann B. Experimental evaluation of surfactants for replacement therapy. Experimental Lung Research 14, 279-310 (1988)). Nur nach Substitution mit Surfactant können diese lungenunreifen Tiere beatmet werden. Durch direkten Vergleich zweier Chargen an Tieren derselben Wurfe wird die relative Potenz bestimmt, das ist die Menge, die die gleiche Wirksamkeit besitzt. In diesem tierexperimentellen Modell zeigte sich, daß der Calciumzusatz im Konzentrationsbereich von 3 mmol/l im Suspendiermittel auf die Wirksamkeit keinen Einfluß hatte (Figur 2). Die relative Potenz war nicht verändert.

In der Figur 1 zeigen die einzelnen Kurven den Verlauf der Viskositätsänderung [mPas] in der Zeit Minuten. Hierbei gehören die einzelnen Kurven zu folgenden Versuchsreihen:

- hoch konzentr. gebundenes $Ca^{2+}$, Suspensionsmedium enthält 3 mmol $Ca^{2+}$, jedoch kein NaCl
- hoch konzentr. gebundenes $Ca^{2+}$, Suspensionsmedium enthält 75 mmol NaCl, jedoch kein $Ca^{2+}$
- hoch konzentr. gebundenes $Ca^{2+}$, Suspensionsmedium enthält 1 mmol $Ca^{2+}$ und 75 mmol NaCl
- hoch konzentr. gebundenes $Ca^{2+}$, Suspensionsmedium enthält 3 mmol $Ca^{2+}$ und 75 mmol NaCl
- nieder konzentr. gebundenes $Ca^{2+}$, Suspensionsmedium enthält 3 mmol $Ca^{2+}$ und 75 mmol NaCl
- nieder konzentr. gebundenes $Ca^{2+}$, Suspensionsmedium enthält 12 mmol $Ca^{2+}$ mit 75 mmol NaCl

EP 0 406 732 B1

Die Figur 2 zeigt, daß der Calciumzusatz im Konzentrationsbereich von 3 mmol/l im Suspendiermittel auf die Wirksamkeit bei unreifen Kaninchenfeten keinen Einfluß hat. Die durchgezogene Gerade gibt die Verhältnisse bei Abwesenheit von Calciumchlorid, die gestrichelt gezeichnete Gerade bei Anwesenheit von 3 mmol $Ca^{2+}$-Ionen (als $CaCl_2$) pro Liter an; aufgetragen ist die gemessene Potenz als Atemzugvolumen, das bei 3 KPa Beatmungsdruck erreicht wird, in Abhängigkeit von log Dosis.

Die nachfolgenden Beispiele beschreiben die Herstellung einiger erfindungsgemäßer Suspensionen:

Beispiel 1

Verwendet wird ein Surfactant, das durch Extraktion aus Rinderlunge mit organischen Lösungsmitteln gewonnen wurde. Es besteht aus etwa 88 % Phospholipiden, 4 % Cholesterol, 1 % Surfactant-assoziierten hydrophoben Proteinen, 0,6 % freien Fettsäuren, sowie Triglyceriden und gebundenem Calcium. Die mittlere relative Molekülmasse der Phospholipide beträgt gerundet 760 D. Die Substanz ist ein gelbes amorphes Pulver unlöslich in Wasser, gut löslich in unpolaren Lösungsmitteln, wie Chloroform; in Wasser kann sie leicht durch Schütteln oder Eintrag von Ultraschallenergie suspendiert werden. Ab einer Konzentration von 5 Gew.-% werden die Suspensionen sichtbar viskos. Die Herstellung, Isolierung und Aufarbeitung erfolgt in Anlehnung an die in Lipids 18: 522-529 (1983) beschriebene Methode unter zusätzlicher Zuführung von $Ca^{2+}$-Ionen. Falls erforderlich, kann der Calciumgehalt auch nachträglich noch im gewünschten Ausmaß korrigiert werden, z. B. durch Zugabe von in Methanol gelöstem Calciumchlorid in der berechneten Menge zu dem in organischem Lösungsmittel, z. B. Chloroform, gelösten Surfactant.

1,5 g des vorbeschriebenen, feinpulvrig gesiebten Surfactants, oder einer durch Gefriertrocknung eines in Tertiärbutanol oder Cyclohexan gelösten Surfactants gewonnenen Substanz werden mit 8,5 ml eines Suspendiermittels, vorzugsweise Kochsalzlösung, versetzt, das 75 mmol/l Kochsalz und 3 mmol/l Calciumchlorid enthält, am besten in einer Injektionsflasche. Die Injektionsflasche wird jetzt geschüttelt und das Surfactant dadurch suspendiert. Die Suspension wird mit einer Injektionskanüle Größe 1 (20 G) in einer Injektionsspritze aufgezogen und anschließend durch eine Kanüle der Größe 18 (26 G) in die Injektionsflasche zurückgespritzt. Das Zurückdrücken in die Injektionsflasche durch die dünne Kanüle erfolgt nur aus Sicherheitsgründen; damit ist sichergestellt, daß die Suspension klumpenfrei ist. Die Applikation der Suspension erfolgt beispielsweise über einen Nabelvenenkatheter in den Trachealtubus des beatmeten Patienten.

Beispiel 2

2,5 g des feinpulvrigen gesiebten Surfactants werden mit 7,5 ml Suspendiermittel (Kochsalzlösung) versetzt, welches 150 mmol/l Kochsalz und 3,5 mmol/l Calciumchlorid enthält. Die weitere Verarbeitung erfolgt wie in Beispiel 1 beschrieben.

**Patentansprüche**
**Patentansprüche für folgende Vertragsstaaten : AT, BE, CH, DE, DK, FR, GB, IT, LI, LU, NL, SE**

1. Verfahren zur Herstellung von Surfactant-Suspensionen zur Substitutionstherapie bei Atemwegserkrankungen, wobei Rinderlungen mit einer Kochsalzlösung ausgewaschen werden, dadurch gekennzeichnet, daß

   a.) das Surfactant aus einer Lösung, die 6 bis 12 mmol/l $Ca^{2+}$ und/oder $Mg^{2+}$-Ionen enthält, abzentrifugiert wird,

   b.) eine erste Reinigung über eine Dichtegradientenzentrifugation mit Glucose in Gegenwart von 3 bis 8 mmol/l $Ca^{2+}$ und/oder $Mg^{2+}$-Ionen erfolgt,

   c.) eine zweite Reinigung durch Verteilung im System organisches Lösungsmittel-Alkohol-Wasser unter Verwendung einer wäßrigen Phase mit 3 bis 10 mmol/l $Ca^{2+}$ und/oder $Mg^{2+}$-Ionen vorgenommen wird,

   d.) die organische Phase anschließend entfernt und der Rückstand getrocknet und zerkleinert wird und

   e.) das so erhaltene feinpulverige trockene Lipid in einer wäßrigen Lösung, die mindestens 1 mmol/l $Ca^{2+}$ und/oder $Mg^{2+}$-Ionen und mindestens 33 mmol/l Kochsalz enthalt, suspendiert wird,

   wobei auch bei Surfactant-Einwaagen von über 60 mg/ml die Viskosität der Suspension unter 30 mPas bleibt.

EP 0 406 732 B1

**2.** Verfahren gemäß Anspruch 1, dadurch gekennzeichnet, daß die durch Auswaschen von Rinderlungen erhaltene Lavage-Flüssigkeit auf einen Gehalt von 6 bis 12 mmol/l $Ca^{2+}$ eingestellt wird.

**3.** Verfahren gemäß Anspruch 1, dadurch gekennzeichnet, daß im Schritt c) die Verteilung im System Chloroform-Methanol-Wasser vorgenommen wird.

**4.** Verfahren gemäß Anspruch 1, dadurch gekennzeichnet, daß im Schritt e) das feinpulvrige trockene Lipid in einer wäßrigen Lösung, die 2,5 bis 3,5 mmol/l $Ca^{2+}$ und/oder $Mg^{2+}$-Ionen und 60 bis 150 mmol/l Natriumchlorid enthält, suspendiert wird.

**5.** Verfahren gemäß Anspruch 1, dadurch gekennzeichnet, daß im Schritt d) das organische Lösungsmittel durch Gefriertrocknung entfernt wird und im Schritt e) die Suspendierung des trockenen Lipids erst nach Bedarf erfolgt.

**6.** Verfahren gemäß Anspruch 1, dadurch gekennzeichnet, daß Surfactant-Suspensionen geringerer Viskosität durch Verdünnen von im Schritt e) erhaltenen Suspensionen mit einem wäßrigen Suspendiermittel, welches mindestens 1 mmol/l $Ca^{2+}$ und/oder $Mg^{2+}$-Ionen und gleichzeitig mindestens 33 mmol/l Natriumchlorid enthält, erhalten werden.

**7.** Verfahren gemäß Anspruch 1, dadurch gekennzeichnet, daß das Surfactant nachträglich in seinem $Ca^{2+}$ und/oder $Mg^{2+}$-Gehalt durch Hinzufügung von deren Salzen in alkoholischer Lösung eingestellt wird.

**8.** Trockenes Lipid, dadurch gekennzeichnet, daß es gemäß den Schritten a) bis d) des Anspruchs 1 hergestellt wird.

**9.** Surfactant-Suspension zur Substitutionstherapie bei Atemwegserkrankungen, erhältlich gemäß Anspruch 1, dadurch gekennzeichnet, daß
   a.) das verwendete Surfactant-Lipid/Protein einen Gehalt an an dieses gebundenen Calcium- und/oder Magnesiumionen von mehr als 25 mmol/mol organisch extrahierbarer Phospholipide,
   b.) das Suspendiermittel mindestens 1 mmol/l Calcium- und/oder Magnesiumionen und
   c.) gleichzeitig mindestens 33 mmol/l Kochsalz enthält,
wodurch auch bei Surfactant-Einwaagen von über 60 mg/ml die Viskosität der Suspension unter 30 mPas bleibt.

**10.** Surfactant-Suspension nach Anspruch 9, dadurch gekennzeichnet, daß das verwendete Surfactant-Lipid/Protein einen Gehalt an gebundenen Calcium- und/oder Magnesiumionen von 40 bis 70 mmol/mol organisch extrahierbarer Phospholipide, das Suspensionsmittel 2,5 bis 3,5 mmol/l Calcium- und/oder Magnesiumionen und 60 bis 150 mmol/l Natriumchlorid enthält.

**11.** Surfactant-Suspension nach Anspruch 9 und 10, dadurch gekennzeichnet, daß das Suspendiermittel Wasser 75 mmol/l Natriumchlorid enthält.

**12.** Surfactant-Suspension nach Anspruch 9, 10 und 11, dadurch gekennzeichnet, daß das Surfactant-Lipid-Protein an dieses gebundene $Ca^{2+}$-Ionen und das Suspendiermittel Wasser Natriumchlorid und Calciumionen enthält.

**Patentansprüche für folgende Vertragsstaaten : ES, GR**

**1.** Verfahren zur Herstellung von Surfactant-Suspensionen zur Substitutionstherapie bei Atemwegserkrankungen, wobei Rinderlungen mit einer Kochsalzlösung ausgewaschen werden, dadurch gekennzeichnet, daß
   a.) das Surfactant aus einer Lösung, die 6 bis 12 mmol/l $Ca^{2+}$ und/oder $Mg^{2+}$-Ionen enthält, abzentrifugiert wird,
   b.) eine erste Reinigung über eine Dichtegradientenzentrifugation mit Glucose in Gegenwart von 3 bis 8 mmol/l $Ca^{2+}$ und/oder $Mg^{2+}$-Ionen erfolgt,
   c.) eine zweite Reinigung durch Verteilung im System organisches Lösungsmittel-Alkohol-Wasser unter Verwendung einer wäßrigen Phase mit 3 bis 10 mmol/l $Ca^{2+}$ und/oder $Mg^{2+}$-Ionen vorgenommen wird,

9

EP 0 406 732 B1

d.) die organische Phase anschließend entfernt und der Rückstand getrocknet und zerkleinert wird und

e.) das so erhaltene feinpulverige trockene Lipid in einer wäßrigen Lösung, die mindestens 1 mmol/l $Ca^{2+}$ und/oder $Mg^{2+}$-Ionen und mindestens 33 mmol/l Kochsalz enthält, suspendiert wird, wobei auch bei Surfactant-Einwaagen von über 60 mg/ml die Viskosität der Suspension unter 30 mPas bleibt.

2. Verfahren gemäß Anspruch 1, dadurch gekennzeichnet, daß die durch Auswaschen von Rinderlungen erhaltene Lavage-Flüssigkeit auf einen Gehalt von 6 bis 12 mmol/l $Ca^{2+}$ eingestellt wird.

3. Verfahren gemäß Anspruch 1, dadurch gekennzeichnet, daß im Schritt c) die Verteilung im System Chloroform-Methanol-Wasser vorgenommen wird.

4. Verfahren gemäß Anspruch 1, dadurch gekennzeichnet, daß im Schritt e) das feinpulvrige trockene Lipid in einer wäßrigen Lösung, die 2,5 bis 3,5 mmol/l $Ca^{2+}$ und/oder $Mg^{2+}$-Ionen und 60 bis 150 mmol/l Natriumchlorid enthält, suspendiert wird.

5. Verfahren gemäß Anspruch 1, dadurch gekennzeichnet, daß im Schritt d) das organische Lösungsmittel durch Gefriertrocknung entfernt wird und im Schritt e) die Suspendierung des trockenen Lipids erst nach Bedarf erfolgt.

6. Verfahren gemäß Anspruch 1, dadurch gekennzeichnet, daß Surfactant-Suspensionen geringerer Viskosität durch Verdünnen von im Schritt e) erhaltenen Suspensionen mit einem wäßrigen Suspendiermittel, welches mindestens 1 mmol/l $Ca^{2+}$ und/oder $Mg^{2+}$-Ionen und gleichzeitig mindestens 33 mmol/l Natriumchlorid enthält, erhalten werden.

7. Verfahren gemäß Anspruch 1, dadurch gekennzeichnet, daß das Surfactant nachträglich in seinem $Ca^{2+}$ und/oder $Mg^{2+}$-Gehalt durch Hinzufügung von deren Salzen in alkoholischer Lösung eingestellt wird.

8. Verfahren zur Herstellung eines trockenes Lipids, dadurch gekennzeichnet, daß es gemäß den Schritten a) bis d) des Anspruchs 1 hergestellt wird.

**Claims**
**Claims for the following Contracting States : AT, BE, CH, DE, DK, FR, GB, IT, LI, LU, NL, SE**

1. Process for preparing surfactant suspensions for replacement therapy in diseases of the respiratory tract, wherein cattle lungs are irrigated with a saline solution, characterised in that

a) the surfactant is centrifuged from a solution containing 6 to 12 mmol/l of $Ca^{2+}$- and/or $Mg^{2+}$-ions,

b) a first purification is carried out by means of density gradient centrifugation with glucose in the presence of 3 to 8 mmol/l of $Ca^{2+}$- and/or $Mg^{2+}$-ions,

c) a second purification is carried out by distribution in a system comprising organic solvent/alcohol/water using an aqueous phase with 3 to 10 mmol/l of $Ca^{2+}$- and/or $Mg^{2+}$-ions,

d) the organic phase is subsequently removed and the residue is dried and comminuted and

e) the finely powdered dry lipid thus obtained is suspended in an aqueous solution containing at least 1 mmol/l of $Ca^{2+}$- and/or $Mg^{2+}$-ions and at least 33 mmol/l of common salt,

the viscosity of the suspension remaining below 30 mPas even with a surfactant content of more than 60 mg/ml.

2. Process according to claim 1, characterised in that the lavage liquid obtained by irrigation of cattle lungs is adjusted to a content of 6 to 12 mmol/l of $Ca^{2+}$.

3. Process according to claim 1, characterised in that, in step c), the distribution in the system of chloroform/methanol/water is carried out.

4. Process according to claim 1, characterised in that, in step e), the finely powdered dry lipid is suspended in an aqueous solution which contains 2.5 to 3.5 mmol/l of $Ca^{2+}$- and/or $Mg^{2+}$-ions and 60 to 150 mmol/l of sodium chloride.

10

EP 0 406 732 B1

5. Process according to claim 1, characterised in that, in step d), the organic solvent is removed after freeze-drying and in step e) suspension of the dry lipid takes place only as necessary.

6. Process according to claim 1, characterised in that surfactant suspensions of lower viscosity are obtained by diluting suspensions obtained in step e) with an aqueous suspending agent which contains at least 1 mmol/l of $Ca^{2+}$- and/or $Mg^{2+}$-ions and at the same time at least 33 mmol/l of sodium chloride.

7. Process according to claim 1, characterised in that the surfactant is subsequently adjusted in its $Ca^{2+}$- and/or $Mg^{2+}$-content by the addition of salts thereof in alcoholic solution.

8. Dry lipid, characterised in that it is prepared according to steps a) to d) of claim 1.

9. Surfactant suspension for replacement therapy in diseases of the respiratory tract, obtainable according to claim 1, characterised in that
a) the surfactant lipid/protein used contains an amount of calcium and/or magnesium ions bound thereto of more than 25 mmol per mol of organically extractable phospholipids,
b) the suspending agent contains at least 1 mmol/l of calcium and/or magnesium ions and
c) at the same time contains at least 33 mmol/l of common salt,
as a result of which the viscosity of the suspension remains below 30 mPas even with surfactant contents of over 60 mg/ml.

10. Surfactant suspension according to claim 9, characterised in that the surfactant lipid/protein used contains an amount of bound calcium and/or magnesium ions of 40 to 70 mmol per mol of organically extractable phospholipids and the suspension agent contains 2.5 to 3.5 mmol/l of calcium and/or magnesium ions and 60 to 150 mmol/l of sodium chloride.

11. Surfactant suspension according to claims 9 and 10, characterised in that the suspending agent, water, contains 75 mmol/l of sodium chloride.

12. Surfactant suspension according to claims 9, 10 and 11, characterised in that the surfactant lipid protein contains $Ca^{2+}$-ions bound thereto and the suspending agent, water, contains sodium chloride and calcium ions.

**Claims for the following Contracting States : ES, GR**

1. Process for preparing surfactant suspensions for replacement therapy in diseases of the respiratory tract, wherein cattle lungs are irrigated with a saline solution, characterised in that
a) the surfactant is centrifuged from a solution containing 6 to 12 mmol/l of $Ca^{2+}$- and/or $Mg^{2+}$-ions,
b) a first purification is carried out by means of density gradient centrifugation with glucose in the presence of 3 to 8 mmol/l of $Ca^{2+}$- and/or $Mg^{2+}$-ions,
c) a second purification is carried out by distribution in a system comprising organic solvent/alcohol/water using an aqueous phase with 3 to 10 mmol/l of $Ca^{2+}$- and/or $Mg^{2+}$-ions,
d) the organic phase is subsequently removed and the residue is dried and comminuted and
e) the finely powdered dry lipid thus obtained is suspended in an aqueous solution containing at least 1 mmol/l of $Ca^{2+}$- and/or $Mg^{2+}$-ions and at least 33 mmol/l of common salt,
the viscosity of the suspension remaining below 30 mPas even with a surfactant content of more than 60 mg/ml.

2. Process according to claim 1, characterised in that the lavage liquid obtained by irrigation of cattle lungs is adjusted to a content of 6 to 12 mmol/l of $Ca^{2+}$.

3. Process according to claim 1, characterised in that, in step c), the distribution in the system of chloroform/methanol/water is carried out.

4. Process according to claim 1, characterised in that, in step e), the finely powdered dry lipid is suspended in an aqueous solution which contains 2.5 to 3.5 mmol/l of $Ca^{2+}$- and/or $Mg^{2+}$-ions and 60 to 150 mmol/l of sodium chloride.

11

**5.** Process according to claim 1, characterised in that, in step d), the organic solvent is removed after freeze-drying and in step e) suspension of the dry lipid takes place only as necessary.

**6.** Process according to claim 1, characterised in that surfactant suspensions of lower viscosity are obtained by diluting suspensions obtained in step e) with an aqueous suspending agent which contains at least 1 mmol/l of $Ca^{2+}$- and/or $Mg^{2+}$-ions and at the same time at least 33 mmol/l of sodium chloride.

**7.** Process according to claim 1, characterised in that the surfactant is subsequently adjusted in its $Ca^{2+}$- and/or $Mg^{2+}$-content by the addition of salts thereof in alcoholic solution.

**8.** Process for preparing a dry lipid, characterised in that it is prepared according to steps a) to d) of claim 1.

**Revendications**

**Revendications pour les Etats contractants suivants : AT, BE, CH, DE, DK, FR, GB, IT, LI, LU, NL, SE**

**1.** Procédé de préparation de suspensions de tensioactif pour une thérapie de substitution en cas de maladies des voies respiratoires, dans lequel des poumons de boeuf sont soumis à une extraction par lavage avec une solution de chlorure de sodium, caractérisé en ce que

a) le tensioactif est séparé par centrifugation d'une solution qui contient 6 à 12 mmol/l d'ions $Ca^{2+}$ et/ou $Mg^{2+}$,

b) une première purification est opérée par centrifugation en gradient de densité avec du glucose en présence de 3 à 8 mmol/l d'ions $Ca^{2+}$ et/ou $Mg^{2+}$,

c) une seconde purification est opérée par partage dans le système solvant organique-alcool-eau au moyen d'une phase aqueuse contenant 3 à 10 mmol/l d'ions $Ca^{2+}$ et/ou $Mg^{2+}$,

d) la phase organique est ensuite éliminée et le résidu est séché et broyé et

e) le lipide sec finement pulvérisé ainsi obtenu est mis en suspension dans une solution aqueuse qui contient au moins 1 mmol/l d'ions $Ca^{2+}$ et/ou $Mg^{2+}$ et au moins 33 mmol/l de chlorure de sodium,

la viscosité de la suspension demeurant inférieure à 30 mPas même dans le cas de pesées de tensioactif supérieures à 60 mg/ml.

**2.** Procédé selon la revendication 1, caractérisé en ce que le liquide de lavage obtenu en soumettant des poumons de boeuf à une extraction par lavage est ajusté à une teneur en $Ca^{2+}$ de 6 à 12 mmol/l.

**3.** Procédé selon la revendication 1, caractérisé en ce que, dans l'étape c), le partage est réalisé dans le système chloroforme-méthanol-eau.

**4.** Procédé selon la revendication 1, caractérisé en ce que, dans l'étape e), le lipide sec finement pulvérisé est mis en suspension dans une solution aqueuse qui contient 2,5 à 3,5 mmol/l d'ions $Ca^{2+}$ et/ou $Mg^{2+}$ et 60 à 150 mmol/l de chlorure de sodium.

**5.** Procédé selon la revendication 1, caractérisé en ce que, dans l'étape d), le solvant organique est éliminé par lyophilisation et, dans l'étape e), la mise en suspension du lipide sec ne se produit que selon les besoins.

**6.** Procédé selon la revendication 1, caractérisé en ce que des suspensions de tensioactif de plus faible viscosité sont obtenues par dilution de suspensions obtenues dans l'étape e) avec un agent de suspension aqueux qui contient au moins 1 mmol/l d'ions $Ca^{2+}$ et/ou $Mg^{2+}$ et en même temps au moins 33 mmol/l de chlorure de sodium.

**7.** Procédé selon la revendication 1, caractérisé en ce que le tensioactif est ajusté ultérieurement en ce qui concerne sa teneur en ions $Ca^{2+}$ et/ou $Mg^{2+}$ par addition de leurs sels en solution alcoolique.

**8.** Lipide sec, caractérisé en ce qu'il est préparé selon les étapes a) à d) de la revendication 1.

**9.** Suspension de tensioactif pour une thérapie de substitution dans le cas de maladies des voies respiratoires, qui peut être obtenue selon la revendication 1, caractérisée en ce que

a) le lipide/protéine tensioactif utilisé a une teneur en ions calcium et/ou magnésium liés à celui-ci supérieure à 25 mmol/mol de phospholipides extractibles par voie organique,
b) l'agent de suspension contient au moins 1 mmol/l d'ions calcium et/ou magnésium et
c) contient en même temps au moins 33 mmol/l de chlorure de sodium,
de sorte que la viscosité de la suspension demeure inférieure à 30 mPas même en cas de pesées de tensioactif supérieures à 60 mg/ml.

10. Suspension de tensioactif selon la revendication 9, caractérisée en ce que le lipide/protéine tensioactif utilisé contient une teneur en ions calcium et/ou magnésium liés de 40 à 70 mmol/mol de phospholipides extractibles par voie organique, l'agent de suspension contient 2,5 à 3,5 mmol/l d'ions calcium et/ou magnésium et 60 à 150 mmol/l de chlorure de sodium.

11. Suspension de tensioactif selon les revendications 9 et 10, caractérisée en ce que l'agent de suspension eau contient 75 mmol/l de chlorure de sodium.

12. Suspension de tensioactif selon les revendications 9, 10 et 11, caractérisée en ce que le lipide/protéine tensioactif contient des ions $Ca^{2+}$ liés à celui-ci et l'agent de suspension eau contient du chlorure de sodium et des ions calcium.

**Revendications pour les Etats contractants suivants : ES, GR**

1. Procédé de préparation de suspensions de tensioactif pour une thérapie de substitution en cas de maladies des voies respiratoires, dans lequel des poumons de boeuf sont soumis à une extraction par lavage avec une solution de chlorure de sodium, caractérisé en ce que
   a) le tensioactif est séparé par centrifugation d'une solution qui contient 6 à 12 mmol/l d'ions $Ca^{2+}$ et/ou $Mg^{2+}$,
   b) une première purification est opérée par centrifugation en gradient de densité avec du glucose en présence de 3 à 8 mmol/l d'ions $Ca^{2+}$ et/ou $Mg^{2+}$,
   c) une seconde purification est opérée par partage dans le système solvant organique-alcool-eau au moyen d'une phase aqueuse contenant 3 à 10 mmol/l d'ions $Ca^{2+}$ et/ou $Mg^{2+}$,
   d) la phase organique est ensuite éliminée et le résidu est séché et broyé et
   e) le lipide sec finement pulvérisé ainsi obtenu est mis en suspension dans une solution aqueuse qui contient au moins 1 mmol/l d'ions $Ca^{2+}$ et/ou $Mg^{2+}$ et au moins 33 mmol/l de chlorure de sodium, la viscosité de la suspension demeurant inférieure à 30 mPas même dans le cas de pesées de tensioactif supérieures à 60 mg/ml.

2. Procédé selon la revendication 1, caractérisé en ce que le liquide de lavage obtenu en soumettant des poumons de boeuf à une extraction par lavage est ajusté à une teneur en $Ca^{2+}$ de 6 à 12 mmol/l.

3. Procédé selon la revendication 1, caractérisé en ce que, dans l'étape c), le partage est réalisé dans le système chloroforme-méthanol-eau.

4. Procédé selon la revendication 1, caractérisé en ce que, dans l'étape e), le lipide sec finement pulvérisé est mis en suspension dans une solution aqueuse qui contient 2,5 à 3,5 mmol/l d'ions $Ca^{2+}$ et/ou $Mg^{2+}$ et 60 à 150 mmol/l de chlorure de sodium.

5. Procédé selon la revendication 1, caractérisé en ce que, dans l'étape d), le solvant organique est éliminé par lyophilisation et, dans l'étape e), la mise en suspension du lipide sec ne se produit que selon les besoins.

6. Procédé selon la revendication 1, caractérisé en ce que des suspensions de tensioactif de plus faible viscosité sont obtenues par dilution de suspensions obtenues dans l'étape e) avec un agent de suspension aqueux qui contient au moins 1 mmol/l d'ions $Ca^{2+}$ et/ou $Mg^{2+}$ et en même temps au moins 33 mmol/l de chlorure de sodium.

7. Procédé selon la revendication 1, caractérisé en ce que le tensioactif est ajusté ultérieurement en ce qui concerne sa teneur en ions $Ca^{2+}$ et/ou $Mg^{2+}$ par addition de leurs sels en solution alcoolique.

13

8.  Procédé de préparation d'un lipide sec, caractérisé en ce qu'il est préparé selon les étapes a) à d) de la revendication 1.

Figur 1

Figur 2

Dosis-Wirkungs-Gerade
Beatmungsdruck konstant 3KPa

ml
Atemzugvolumen